# NEW EUROPEAN PATENT SPECIFICATION

(11) **EP 2 177 501 B3**
(45) Date of publication of this specification: **02.11.2016**
(45) Mention of the grant of the patent: 05.11.2014
(21) Application number: 09171245.5
(22) Date of filing: 24.09.2009
(51) Int. Cl.: C07C 213/04, C07C 215/08, C07C 215/12, B01J 19/00

(54) **Method and plant for the production of ethanol amines**
Verfahren und Anlage zur Herstellung von Ethanolaminen
Procédé et installation pour la production d'amines d'éthanol

(30) Priority: 20.10.2008 EP 08167010
(43) Date of publication of application: 21.04.2010
(73) Proprietor: Sulzer Chemtech AG, 8404 Winterthur (CH)
(72) Inventor: Fässler, Peter, 8400 Winterthur (CH)
(74) Representative: Manitz, Finsterwald & Partner GbR

(56) References cited:
- EP-A- 1 219 592
- EP-A- 1 443 036
- US-A- 4 169 856
- US-A- 4 355 181

## Description

The invention relates to a method and a plant for the production of ethanolamines

Ethanolamines are flammable, corrosive, colorless, viscous liquids that are produced by the reaction between ammonia (NH₃) and ethylene oxide (EO). Identified many years ago, ethanolamines are a key ingredient in a number of important product formulations such as cosmetics and personal hygiene applications, agricultural products, wood-preservation chemicals, soaps and detergents, gas treatments. They can also be used in the production of nonionic detergents, emulsifiers, and soaps, as well as in emulsion paints, polishes, and cleansers.

There are 3 types of ethanolamines: monoethanolamines (MEA), diethanolamines (DEA), and triethanolamines (TEA). The formation of MEA, DEA, or TEA depends on whether an ammonia molecule reacts with one, two, or three ethylene oxide molecules.

The volume of water used in ethanolamine production can vary: if the water content remains low during the process, it is necessary for the reaction to be conducted under high pressure, thus resulting in high investment costs. These reactions consume a low level of energy. In order to decrease investment costs, a high water content may be used under low pressure, but the related energy consumption will consequently be higher. Such a low pressure process is for instance disclosed in EP1219592 A1 in which it is disclosed that the operating pressure of the ammonia stripper is about 2 bars. The operating pressure of the process according to US4169856 is even lower to be at a maximum of 0,001 bar. Another approach is to remove low boiling substances, such as unreacted ammonia and water from an intermediate product, which is then further treated in a multistage vacuum distillation. Finally the distillate to of the vacuum distillation is subjected to a further distillation step. Such a process is perfectly suitable in a laboratory scale for a feed of 0.1 liter/hr, however the energy consumption of the distillation steps would increase considerably if the process was applied industrially.

There is a need for a process for the production of ethanol amines, which overcomes the shortcomings of the prior art ethanol amine processes, thus which can be operated with lower energy consumption.

It is an object of the invention to provide a process and a plant for the production of ethanol amine, for which the amount of water to be vaporised is decreased with respect to the prior art.

The object is solved by a method for the production of ethanol amines, comprising the steps of adding ammonia, ethylene oxide and water to a reactor, whereby in the reactor, the ethylene oxide is reacted to a reaction product comprising at least one of a monoethanolamine, a diethanolamine and a triethanolamine, stripping the ammonia from the reaction product in an ammonia stripper, concentrating the reaction product by evaporating water and feeding the concentrated reaction product to a distillation plant for separating the triethanolamine from the monoethanolamine and diethanolamine, characterised in that the ammonia stripped from the reaction product is fed to an ammonia absorber for recycling the ammonia to the reactor, wherein a portion of reaction product to be fed to the ammonia stripper is heated by means of a heat exchanger with a vapor from the evaporator, and wherein the ammonia stripper is integrated directly with the ammonia absorber by feeding a stripper overhead vapour into a bottom of the ammonia absorber.

Advantageously the ammonia absorber operates at a pressure of above 4 bar, preferably at or above 4.5 bar, particularly preferred at or above 4.8 bar. This operation under a higher pressure increases the ammonia concentration in the feed stream to the reactor. Thus, the ammonia absorber has a sump, wherein the ammonia concentration in the sump is at least 40 %, particularly preferred at least 42.3%.

The method results in a good product quality, which can be expressed by the degree of coloring of the MEA, DEA and TEA products. A lower color value consequently leads to higher product quality. The colors of the MEA and DEA leaving the distillation plant are below 10.

The ammonia to ethanolamine ratio is up to or exceeding 6:1 when entering the reactor. By variation of the ammonia to ethanolamine ratio, the product distribution can be shifted, for instance to preferably higher DEA and/or MEA values.

The concentrated reaction product has a maximum temperature of 180°C before entering the distillation plant. This limitation reduces the thermal stress on the products, thus a degradation to higher color values. The temperature in the reactor is decreased for lowering the concentration of triethanolamines.

A plant for the production of ethanol amines comprises a reactor, whereby in the reactor, ethylene oxide is reacted with ammonia and water to a reaction product comprising a monoethanolamine, diethanolamine and triethanolamine. The plant further comprises an ammonia stripper for stripping the ammonia from the reaction product, an evaporator for evaporating water whereby a concentrated reaction product is obtainable and a distillation plant for separating the concentrated reaction product containing the triethanolamine from the monoethanolamine and diethanolamine. An ammonia absorber is foreseen for recycling the ammonia stripped from the reaction product to the reactor and wherein the ammonia stripper is integrated directly with the ammonia absorber by means of a direct connection between them.

A heat exchanger is advantageously foreseen for heating a portion of stripped reaction product to be fed to the ammonia stripper with a vapor from the evaporator. The heat exchanger comprises a fall film evaporator, a thermosiphon or a forced circuit reboiler.

The distillation plant in a preferred variant comprises a dehydration column for separating residual water from the concentrated reaction product, wherein a first distillation column is provided for separation of monoethanolamine from diethanolamine and triethanolamine, a second distillation column is provided for separating diethanolamine from triethanolamine.

A third distillation column can be provided for further concentration of the triethanolamine. This third distillation column may comprise a single bed of packings. The triethanolamine leaving the sump of the third distillation column as triethanolamine residue can be further purified in a short path evaporator into a triethanolamine distillate of a color value of less than 40 and a triethanolamine residue.

At least a second reactor can be provided for adding further ethylene oxide to the concentrated reaction product to shift the product distribution to higher portions of diethanolamine.

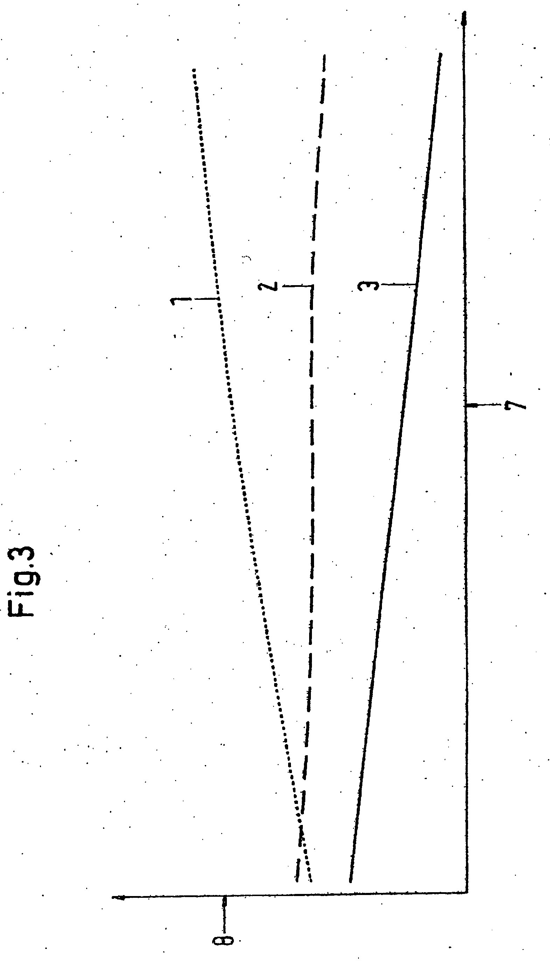

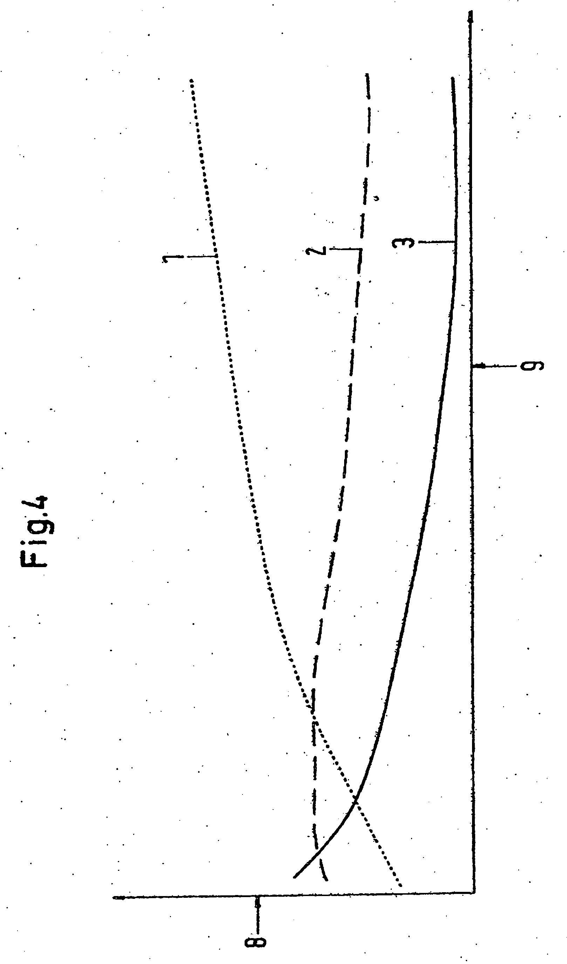

Unlike conventional ethanolamine production technology, which is often limited in terms of the ratio of ammonia to ethanolamine that can be handled, the inventive solution offers the option of altering the amounts of the final product generated. The composition of the resulting mixture is determined by the ratio between the raw materials, which can be varied in accordance with requirements. The higher the proportion of ammonia, the more monoethanolamine is formed (Fig.3). The process according to the invention can handle ammonia to ethanolamine ratios up to or exceeding 6:1 and offers a high level of MEA or DEA distribution while minimizing the volume of TEA produced (Fig.4).

The new ethanolamine technology solution according to the invention combines the advantages of the high-pressure ammonia route with the benefits of the low-pressure process. The process allows the use of a relatively low-pressure reaction and incorporates very advanced heat integration, which saves costs across the board.

The water recovery system, which uses the energy integration concept, thermally ties the water evaporator to the ammonia stripper column and minimizes the size of the drying column. It thus enables the plant to operate economically even at ammonia to ethylene oxide ratios of 6:1 or above. Furthermore, the presence of water as a catalyst in the synthesis of ethanolamine ensures a rational and economical reactor setup. The use of water provides the greatest control over the temperature rise in the reactor system and allows a reduction in the volume of the reactor. The average reaction temperature can be lowered and adjusted to minimize secondary side reactions that could compromise the quality of the resulting ethanolamine.

The key to saving energy in the production of ethanolamines lies in the design and operating pressure of the main ammonia absorber. The ammonia stripper is integrated directly with the absorber by feeding the stripper overhead vapors into the bottom of the ammonia absorber. In other words, there is a direct connection between the two columns. In addition, the overhead vapors from the main water distillation columns are used as a heat source for the ammonia stripper reboiler.

The pressure at the top of the main water evaporation column is limited by the maximum permissible temperature at the bottom of the column. Hence, the boundary operating conditions of the ammonia absorber, ammonia stripper, and main water evaporation column are basically fixed. The only degree of flexibility in the design is in the selection of the column operating conditions, column internals, and the heat exchanger.

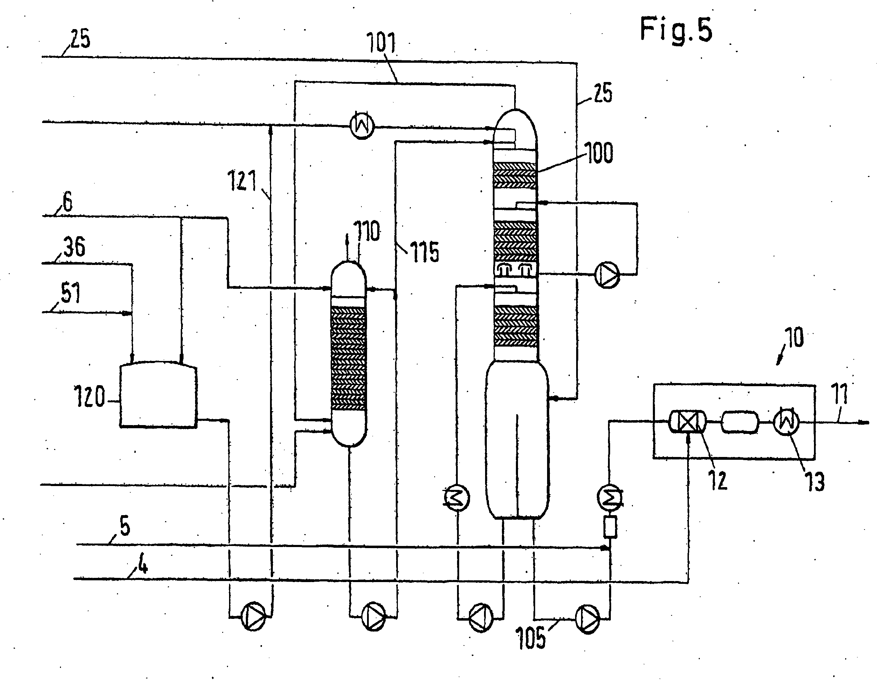

The thermal and energy integration are added to the ammonia absorber, the ammonia stripper, and the water distillation column. As a direct consequence of this thermal and process heat integration, the energy required by the improved process is comparable to the high-pressure route (Fig.5).

The increase in the ammonia concentration in the ammonia absorber is a direct result of the process heat integration and has a significant impact on overall energy consumption. The higher the operating pressure in the ammonia absorber, the higher the ammonia concentration. The higher the ammonia content, the less water has to be handled in the system. Since the water must be recycled back into the system by means of evaporation, each percentage point increase in the ammonia concentration leads to an approximate energy saving of between 1.5 and 2%, which represents a significant reduction in absolute terms.

The ammonia concentration can also be set to allow for the efficient and economical recovery of the residual ammonia from the reaction loop. This recovery occurs at a pressure that is sufficiently low to ensure that the ammonia stripping takes place at a low temperature, thus reducing the possible degradation of the product and minimizing the corrosion of equipment.

The high ammonia concentration allowed by the process according to this invention further reduces the size of the ammonia stripper and ammonia scrubber and eliminates the need for an additional auxiliary reboiler heated by an external heating source. These features significantly reduce investment costs.

Ethanolamines are sensitive to heat and therefore tend to degrade and must be separated with great care. The higher the temperature and the pressure, the more colored the ethanolamines become. Color is, however, the most important characteristic determining their quality and value; the more colorless the substances are, the higher their market value. The use of high-efficiency packings as internals of the columns and the rational design of the distillation train minimizes the packing volume and enables the column to be operated at a lower temperature, with narrow residence time distribution and minimum permanent hold-up. The separation of ethanolamines at these carefully maintained conditions improves the product color and reduces the corrosion at the bottom of the column.

The ethanolamines produced are often recycled back into the main reactor system in order to adjust the desired MEA:DEA:TEA ratio. If the reaction is performed in the primary reactor, the recycling not only consumes more energy and requires a higher residence time but can also lower the product quality through the development of color and an increase in corrosion. The inventive concept avoids the difficulties associated with recycling and eliminates repeated process steps, thus reducing the overall residence time in the plant and lowering energy consumption.

Product stress is defined as the tendency of the product to degrade and carbonize at the bottom of the column. Product stress can be minimized through the rational study of the temperature profile and the configuration at the bottom of the column, resulting in less product degradation. Side reactions, such as color precursors, can be suppressed to a certain extent through the smooth execution of the process at the lowest temperatures possible.

The level of product stress is directly related to the hot product residence time and is exponentially related to the temperature of the hot product. The residence time is equal to the time in minutes during which the average bottom product molecule stays in the bottom of the column. The temperature T is equal to the absolute temperature of the hot stream at the bottom of the column. A is a proportionality factor. The process for ethanolamine production according to the invention makes effective use of the product stress concept to ensure the best handling of the hot product at the bottom of the column.

The synthesis and purification of ethanolamines is a relatively complex process as the final product quality is determined by the presence of minor impurity streams in the feed, as well as by internal recycle streams, thermal process integration and the reaction of individual components including side reactions.

A fully integrated approach throughout the plant is a prerequisite for a successful design. For the design of the method and the plant according to the invention a single block for the entire process simulation has been adopted. This module has been systematically improved not only by integrating the accumulated know-how but also by relying on real plant operating data and performance characteristics. This type of comprehensive simulation tool is sophisticated enough to provide a satisfactory description of plant details and serves as a powerful tool for the analysis of possible process improvements, for heat integration, and for new plant designs. Any adjustments that are required, e.g. relating to product capacity and product distribution, can be implemented relatively quickly and with a high degree of accuracy.

In addition, the accumulation of unwanted byproducts or feed impurity products trapped in the process loops can be detected and eliminated through purging at specific locations.

The invention will be explained in the following with reference to the drawings.
Fig. 1 shows the reaction sequence
Fig. 2 shows a scheme of the process according to the invention
Fig. 3 is a diagram showing the product composition based on the amount of ammonia
Fig. 4 is a diagram showing the product composition based on the ratio of MEA/EO
Fig. 5 shows of the ammonia absorber and the reactor of Fig. 2
Fig. 6 shows the ammonia stripper and the evaporator of Fig. 2
Fig. 7 shows a possible variant of the distillation plant of Fig. 2

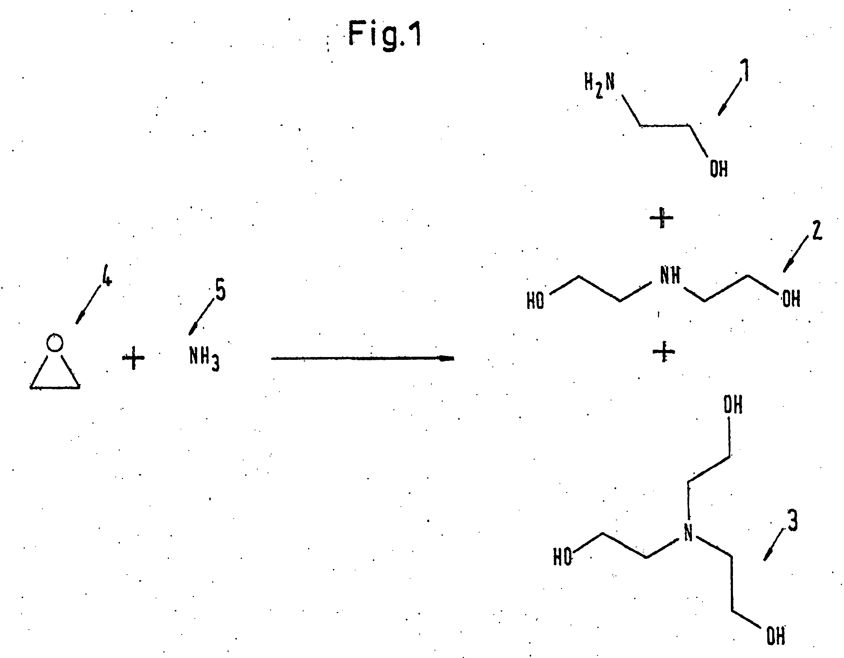

Fig. 1 shows the reaction sequence to obtain monoethanolamines (MEA) 1, diethanolamines (DEA) 2 and triethanolamines (TEA) 3 from a mixture of ethylene oxide (EO) 4 and ammonia (NH₃) 5.

The reaction takes place in an aqueous environment, thus in the presence of water.

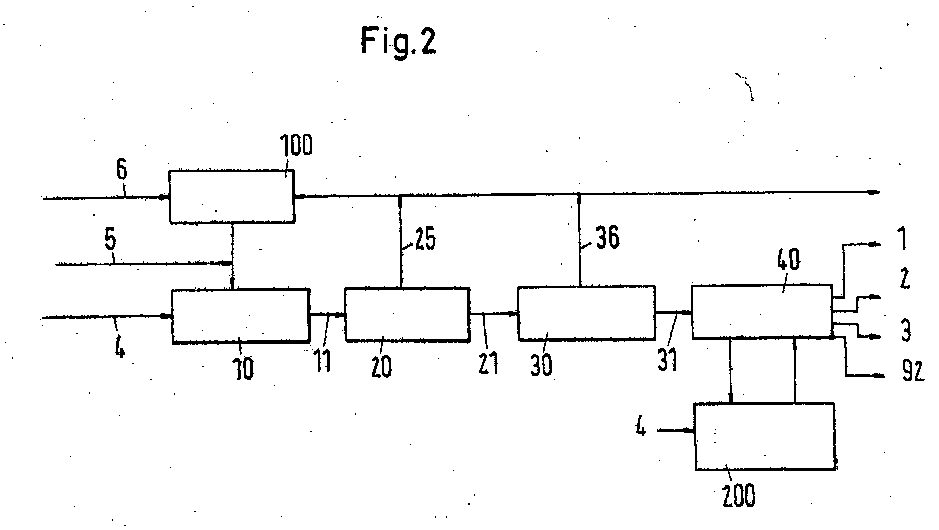

The process for the production of ethanol amines is schematically shown in Fig. 2, that is MEA 1, DEA 2 and TEA 3, comprises the steps of adding ammonia 5, ethylene oxide (EO) 4 and demineralised water 6 to a reactor 10.

The ethylene oxide 4 is reacted to a reaction product 11 comprising MEA 1, DEA 2 and TEA 3. The reaction product 11 enters an ammonia stripper 20 for stripping the ammonia 25 from the reaction product 11. Thereby a stripped reaction product 21 is generated. The stripped reaction product 21 enters an evaporator 30 for concentrating the stripped reaction product 21 by evaporating water to obtain vapor 36. The concentrated reaction product 31 is then fed into a distillation plant 40 for separating the triethanolamines TEA 3 from the monoethanolamines MEA 1 and from the diethanolamines DEA 2. The ammonia 25 stripped from the reaction product 11 is fed to an ammonia absorber 100 for recycling the ammonia 105 to the reactor 10.

A further reactor 200 can be provided in combination with the distillation plant 40 to add ethylene oxide 4 to any of the intermediates produced in at least one of the distillation steps performed in the distillation plant.

Fig. 3 shows the ethanolamine (EA) product distribution according to the reaction performed in reactor 1. The horizontal axis indicates the ratio of ammonia to ethylene oxide 7. For this particular embodiment, the ratio of ammonia to ethylene oxide is in the range from 3 to 6. The vertical axis indicates the weight percent of amines 8, thus of each of the three components MEA 1, DEA 2 and TEA 3 in the reaction product 11. The reaction product 11 additionally contains ammonia and water. For the purposes of Fig. 3 ammonia and water can be disregarded as Fig. 3 only outlines the influence on the composition of the reaction mixture on the reaction product. The composition of the reaction mixture is determined by the ratio of ammonia to ethylene oxide 7. The higher the proportion of ammonia, the more monoethanolamine is formed, as indicated by the curve for MEA 1. The process according to the invention can handle ammonia to ethylene oxide ratios up to or exceeding 6:1 and offers a high level of MEA 1 or DEA 2 distribution while minimizing the volume of TEA 3 produced.

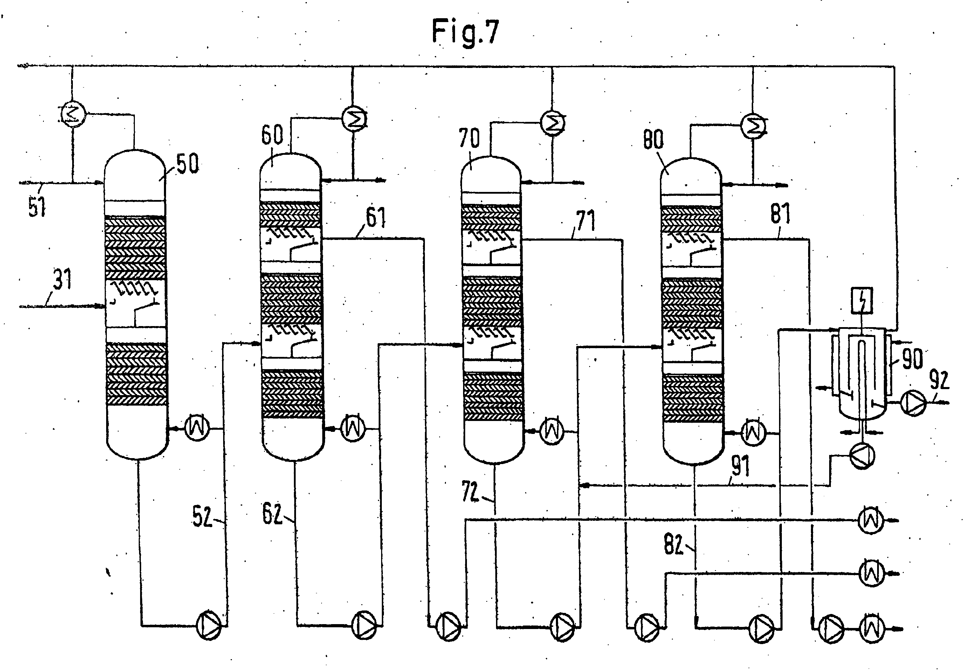

Fig. 4 shows the ethanolamine (EA) product distribution according to a reaction performed in reactor 200. The reaction in reactor 200 is important if the portion of DEA is to be increased. The reactor 200 is arranged in an MEA product stream, such as the product stream 61 of a first distillation column 60 for separation of MEA 1 from DEA 2 and TEA 3. The first distillation column 60 is shown in Fig. 7 in more detail. The horizontal axis of Fig. 4 indicates the ratio of MEA to ethylene oxide 9. In particular, the ratio of MEA to ethylene oxide 9 is in the range from 1 to 5. The vertical axis indicates the weight percent of amines 8, thus of each of the three components MEA, DEA and TEA in the reaction mixture. The higher the proportion of MEA, the more monoethanolamine is formed, as indicated by the curve for MEA 1. Therefore the process according to the invention offers a high level of MEA 1 or DEA 2 distribution while minimizing the volume of TEA 3 produced, which is an unexpected increase in product quality. Thus the amount of TEA can be reduced to a very low level, which has not been possible with conventional low pressure ethanolamine plants. This surprising effect can be attributed to the improved heat exchange, made possible by the introduction of heat exchanger 22, such as a forced circuit reboiler or a fall film evaporator which allows to achieve a higher sump temperature in the ammonia stripper 20. Thereby the percentage of ammonia to be recovered from the stripper is increased and this ammonia 25 is recycled to the ammonia absorber 100. By only a slight increase of the pressure of the ammonia absorber to above 4 bar, in particular to about 4.8 bar, the ammonia concentration in the sump of the ammonia absorber can be increased to 40 % or more, in particular to at least 42.3 percent.

Fig. 5 shows the part of the plant for performing the process for the production of ethanol amines which is related to the equipment for performing the reaction in reactor 10. That means the reaction according to Fig. 1 leading to MEA , DEA and TEA which results in a reaction product 11, containing MEA, DEA and TEA as well ammonia and water. The reactor 10 can for instance be a multi point injection reactor comprising a mixer 12, and an intercooler 13. Ammonia 5, ethylene oxide (EO) 4 and demineralised water 6 are fed to the reactor 10. The ethylene oxide 4 is reacted to a reaction product 11 comprising MEA 1, DEA 2 and TEA 3.

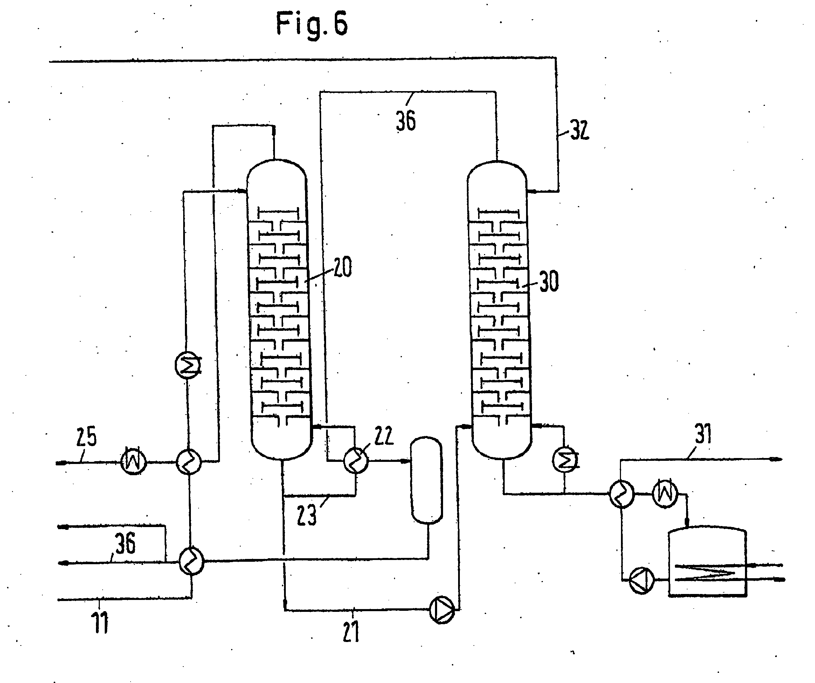

The reaction product 11 enters the ammonia stripper 20 for stripping the ammonia 25 from the reaction product 11, which is shown in Fig. 6. The ammonia 25 from the ammonia stripper 20 is introduced into the ammonia absorber 100 shown in Fig. 5. The sump product of the ammonia absorber is the recycle ammonia 105. Recycle ammonia 105 is mixed with ammonia 5 and enters the reactor 10. If the pressure in the ammonia absorber increases, the higher is the resulting concentration of ammonia in the ammonia 105 stream. The higher the concentration of ammonia, the less water has to be added to the reactor and the less water has to be separated from the reaction product 11 at a later stage.

In Fig. 5 an additional absorber 110 is shown. This absorber 110 is used for recovering any ammonia from other sources, such as tanks, loading station, vacuum pumps and the like. Furthermore the head product 101 of ammonia absorber 100 is fed into this absorber. The ammonia stream 115 leaving ammonia absorber 110 is fed into the main ammonia absorber 100.

Additionally a storage tank 120 is shown for process water, that is in part condensed water vapor 36 from the evaporator 30, and in part water stemming from the distillation plant. From the storage tank 120 a mix of fresh demineralised water 6 and recycle water is fed as product stream 121 to the ammonia absorber 100 or as make up recycle water 32 to the evaporator 30 as shown in Fig. 6.

Fig. 6 shows the plant for performing process steps downstream of the reactor 10. The reaction product 11 is fed into the ammonia stripper 20. Thereby a stripped reaction product 21 is generated. The stripped reaction product 21 enters the evaporator 30. In the evaporator 30, the stripped reaction product 21 is concentrated by evaporating water which leaves the evaporator as vapor 36. The concentrated reaction product 31 is then fed into a distillation plant 40 for separating the triethanolamines TEA 3 from the monoethanolamines MEA 1 and from the diethanolamines DEA 2. The ammonia 25 stripped from the reaction product 11 is fed to an ammonia absorber 100 for recycling the ammonia 105 to the reactor 10. An additional make up recycle water stream 32 is added to evaporator 30 to ensure that no MEA; DEA or TEA will leave the evaporator together with the vapor 36 and to ensure that a mass transfer is possible on all trays of evaporator 30. In particular if the feed of stripped reaction product 21 enters the evaporator close to the bottom thereof, the vapor rising from the sump would not be contacted with liquid descending the trays of the column above the level of the feed. Therefore the make up recycle water 32 is added to the head of evaporator 30

The vapor 36 leaving evaporator 30 is used for heating the portion of the stripped reaction product 23, which is fed back into the ammonia stripper 20. A heat exchanger 22 is provided for heating the portion of stripped reaction product 23. Advantageously the heat exchanger 22 is a forced circuit reboiler, a thermosiphon or a fall film evaporator, which provides the advantages mentioned earlier. Surprisingly the pressure in the ammonia stripper can be increased by foreseeing the heat exchanger 22. The increase of pressure in the ammonia stripper leads to an increase in ammonia concentration, thus an improved stripping result. Due to the fact that the ammonia leaving the ammonia stripper as ammonia stream 25 bound for the ammonia absorber 100, the ammonia concentration in the sump of ammonia absorber 100 can be increased to at least 40 %, particularly preferred at least 42.3 %. Each percent of higher ammonia concentration results in an overall energy saving in a range of 1.5 to 2%. A further advantage associated with heat exchanger 22, in particular of a heat exchanger of one of the types mentioned previously is the possibility to decrease the operating temperature of a step of separating any water still present in the concentrated reaction product as will be described in Fig. 7 for a preferred embodiment.

Fig. 7 shows the further treatment of the concentrated reaction product 31 after having left the evaporator 30. The concentrated reaction product enters the dehydration column 50 for separating any water still present in the concentrated reaction product 31 and recycle such water 51 back to the water storage tank 120. The dewatered reaction product 52 is fed into a first distillation column 60 for separating MEA leaving as MEA product stream 61 from the DEA and TEA stream 62.

The DEA and TEA stream 62 is pumped into a second distillation column 70 for separating DEA and TEA. The DEA leaves the second distillation column as DEA distillate 71. The TEA is concentrated in the sump. This sump product contains TEA of different grades. These different grades of TEA can be distinguished by their color. A TEA with a color grade of a maximum of 40 according to the standard method of APHA is considered as a product suitable for storing or further treatment, whereas TEA with color values above 40 are considered as residue.

The TEA raw product stream 72 can therefore further be purified in a third distillation column 80 used for the separation of TEA from residual TEA. The TEA product stream 81 can be stored and is suitable for processing, whereas the sump product of the TEA column is a TEA residue 82, which contains is further separated in a short path evaporator 90. The TEA distillate 91 of short path evaporator 90 is recycled back into the TEA product stream 72 to be fed into the third column 80. The TEA distillate 91 has a color grade less than 40, whereas the TEA residue leaving the short path evaporator 90 is a viscous pitch of black color. The color values of TEA with an overall plant yield of around 94 % were ranging between 50 and 80.

The MEA and DEA products had in all cases a color value of less than 10, the product purities laid within the specifications and were usually higher than design, in particular the DEA purity was always higher than design.

## Claims

1. A method for the production of ethanol amines, comprising the steps of adding ammonia, ethylene oxide and water to a reactor, whereby in the reactor, the ethylene oxide is reacted to a reaction product comprising a monoethanolamine, a diethanolamine and a triethanolamine, stripping the ammonia from the reaction product in an ammonia stripper, whereby a stripped reaction product is obtained, concentrating the stripped reaction product by evaporating water in an evaporator, whereby a concentrated reaction product is obtained, and feeding the concentrated reaction product to a distillation plant for separating the triethanolamine from the monoethanolamine and diethanolamine, **characterised in that** the ammonia stripped from the reaction product is fed to an ammonia absorber for recycling the ammonia to the reactor, wherein a portion of reaction product to be fed to the ammonia stripper is heated by means of a heat exchanger with a vapor from the evaporator, and wherein the ammonia stripper is integrated directly with the ammonia absorber by feeding a stripper overhead vapour into a bottom of the ammonia absorber.

2. The method according to claim 1, wherein the ammonia absorber operates at a pressure of above 4 bar, preferably at or above 4.5 bar, particularly preferred at or above 4.8 bar.

3. The method according to any of claims 1 or 2, wherein the ammonia absorber has a sump, wherein the ammonia concentration in the sump is at least 40 %, particularly preferred at least 42.3%.

4. The method according to any of the preceding claims wherein the colors of the MEA and DEA leaving the distillation plant are below 10.

5. The method according to any of the preceding claims, wherein the ammonia to ethanolamine ratio is up to or exceeding 6:1 when entering the reactor.

6. The method according to any one of the preceding claims, wherein the concentrated reaction product has a maximum temperature of 180°C before entering the distillation plant.

7. The method according to any one of the preceding claims, wherein the temperature in the reactor is decreased for lowering the concentration of triethanolamines.

8. A plant for the production of ethanol amines comprising a reactor (10), whereby in the reactor, ethylene oxide (4) is reacted with ammonia (5,105) and water (6) to a reaction product (11) comprising a monoethanolamine, diethanolamine and triethanolamine, an ammonia stripper (20) for stripping the ammonia from the reaction product (11), whereby a stripped reaction product (23) is obtained, an evaporator (30) for evaporating water from the stripped reaction product (23) whereby a concentrated reaction product (31) is obtained, and a distillation plant (4) for separating the concentrated reaction product (31) containing the triethanolamine from the monoethanolamine and diethanolamine, whereby an ammonia absorber (100) is foreseen for recycling the ammonia (25) stripped from the reaction product (11) to the reactor (10) **characterised in that** a heat exchanger (22) is provided for heating a portion of stripped reaction product (23) to be fed to the ammonia stripper (20) with a vapor from the evaporator (30), and wherein the ammonia stripper (20) is integrated directly with the ammonia absorber (100) by means of a direct connection between them.

9. The plant according to claim 8 wherein the heat exchanger (22) comprises a fall film evaporator, a thermosiphon or a forced circuit reboiler.

10. The plant according to any of the preceding claims 8 to 9, wherein the distillation plant (40) comprises a dehydration column (50) for separating residual water from the concentrated reaction product (31), wherein a first distillation column (60) is provided for separation of monoethanolamine from diethanolamine and triethanolamine, a second distillation column (70) is provided for separating diethanolamine from triethanolamine.

11. The plant according to claim 10, wherein a third distillation column (80) is provided for further concentration of the triethanolamine.

12. The plant according to claim 11, wherein the third distillation column (80) comprises a single bed of packings.

13. The plant according to claims 10 or 11, wherein the triethanolamine leaving the sump of the third distillation column (80) as triethanolamine residue (82) is further purified in a short path evaporator (90) into a triethanolamine distillate (91) of a color value of less than 40 and a triethanolamine residue (92).

14. The plant according to any of claims 10 to 13, wherein at least a second reactor (200) is provided for adding further ethylene oxide to the concentrated reaction product (31) to shift the product distribution to higher portions of diethanolamine.

## Patentansprüche

1. Verfahren zur Herstellung von Ethanolaminen umfassend die Schritte der Zugabe von Ammoniak, Ethylenoxid und Wasser zu einem Reaktor, wodurch das Ethylenoxid in dem Reaktor zu einem Reaktionsprodukt, welches ein Monoethanolamin, ein Diethanolamin und ein Triethanolamin enthält, reagiert wird, die Strippung des Ammoniaks aus dem Reaktionsprodukt in einem Ammonikstripper, wodurch ein gestripptes Reaktionsprodukt erhalten wird, das Konzentrieren des gestrippten Reaktionsprodukts durch Verdampfen von Wasser in einem Verdampfer, wodurch ein konzentriertes Reaktionsprodukt erhalten wird, und das Zuführen des konzentrierten Reaktionsprodukts zu einer Destillationsanlage zum Abtrennen des Triethanolamins von dem Monoethanolamin und Diethanolamin, **dadurch gekennzeichnet, dass** das aus dem Reaktionsprodukt gestrippte Ammoniak zum Wiederverwerten des Ammoniaks in dem Reaktor zu einem Ammoniakabsorber geleitet wird, wobei ein Teil des Reaktionsproduktes, welches dem Ammoniakstripper zuzuführen ist, mittels eines Wärmeaustauschers mit Dampf aus dem Verdampfer erhitzt wird, und wobei der Ammoniakstripper mit dem Ammoniakabsorber direkt integriert wird, indem ein Dampf der Kopffraktion des Strippers in einen Boden des Ammonikabsorbers eingeleitet wird.

2. Verfahren nach Anspruch 1, wobei der Ammoniakabsorber bei einem Druck von oberhalb von 4 bar, vorzugsweise bei oder oberhalb von 4,5 bar und besonders bevorzugt bei oder oberhalb von 4,8 bar betrieben wird.

3. Verfahren nach Anspruch 1 oder 2, wobei der Ammoniakabsorber einen Sumpf aufweist, wobei die Ammoniakkonzentration in dem Sumpf wenigstens 40 % und besonders bevorzugt wenigstens 42,3 % beträgt.

4. Verfahren nach einem der vorhergehenden Ansprüche, wobei die Farben des MEA und DEA, welche die Destillationsanlage verlassen, weniger als 10 betragen.

5. Verfahren nach einem der vorhergehenden Ansprüche, wobei das Verhältnis Ammoniak zu Ethanolamin beim Eintritt in den Reaktor bis zu oder mehr als 6:1 beträgt.

6. Verfahren nach einem der vorhergehenden Ansprüche, wobei das konzentrierte Reaktionsprodukt vor dem Eintritt in die Destillationsanlage eine maximale Temperatur von 180 °C aufweist.

7. Verfahren nach einem der vorhergehenden Ansprüche, wobei die Temperatur in dem Reaktor zum Verringern der Konzentration an Triethanolaminen verringert wird.

8. Anlage zum Herstellen von Ethanolaminen umfassend einen Reaktor (10), wobei in dem Reaktor Ethylenoxid (4) mit Ammoniak (5, 105) und Wasser (6) zu einem Reaktionsprodukt (11) reagiert wird, Monoethanolamin, Diethanolamin und Triethanolamin enthält, einen Ammoniakstripper (20) zum Strippen des Ammoniaks aus dem Reaktionsprodukt (11), wodurch ein gestripptes Reaktionsprodukt (23) erhalten wird, einen Verdampfer (30) zum Verdampfen von Wasser aus dem gestrippten Reaktionsprodukt (23), wodurch ein konzentriertes Reaktionsprodukt (31) erhalten wird, und eine Destillationsanlage (4) zum Abtrennen des konzentrierten Reaktionsprodukts (31), welches das Triethanolamin enthält, von Monoethanolamin und Diethanolamin, wobei ein Ammoniakabsorber (100) zum Wiederverwerten des aus dem Reaktionsprodukt (11) gestrippten Ammoniaks in dem Reaktor (10) vorgesehen ist, **dadurch gekennzeichnet, dass** ein Wärmeaustauscher (22) zum Erhitzen eines Teils des gestrippten Reaktionsprodukts (23), das dem Ammoniakstripper (20) zuzuführen ist, mit Dampf von dem Verdampfer (30) vorgesehen ist, und wobei der Ammoniakstripper (20) mit dem Ammoniakabsorber (100) mittels einer direkten Verbindung zwischen diesen direkt integriert ist.

9. Anlage nach Anspruch 8, wobei der Wärmeaustauscher (22) einen Dünnfilmverdampfer, einen Thermosiphon oder einen Verdampfer mit erzwungenem Kreislauf umfasst.

10. Anlage nach einem der vorhergehenden Ansprüche 8 oder 9, wobei die Destillationsanlage (40) eine Dehydratisierungssäule (50) zum Abtrennen von restlichem Wasser aus dem konzentrierten Reaktionsprodukt (31) umfasst, wobei eine erste Destillationssäule (60) zur Abtrennung des Monoethanolamins von dem Diethanolamin und Triethanolamin vorgesehen ist und eine zweite Destillationssäule (70) zum Abtrennen von Diethanolamin von Triethanolamin vorgesehen ist.

11. Anlage nach Anspruch 10, wobei eine dritte Destillationssäule (80) zur weiteren Konzentrierung des Triethanolamins vorgesehen ist.

12. Anlage nach Anspruch 11, wobei die dritte Destillationssäule (80) ein Packungseinzelbett umfasst.

13. Anlage nach Anspruch 10 oder 11, wobei das Triethanolamin, welches den Sumpf der dritten Destillationssäule (80) als Triethanolaminrest (82) verlässt, in einem Kurzwegverdampfer (90) zu einem Triethanoldestillat (91) mit einem Farbwert von weniger als 40 und einem Triethanolaminrest (92) weiter gereinigt wird.

14. Anlage nach einem der Ansprüche 10 bis 13, wobei wenigstens ein zweiter Reaktor (200) zur Zugabe von weiterem Ethylenoxid zu dem konzentrierten Reaktionsprodukts (31) vorgesehen ist, um die Produktverteilung zu höheren Anteilen an Diethanolamin zu verschieben.

## Revendications

1. Procédé de production d'éthanol amines, comprenant les étapes consistant à ajouter de l'ammoniac, d'éthylène oxyde et de l'eau à un réacteur, moyennant quoi, dans le réacteur, l'éthylène oxyde est amené à réagir à un produit de réaction comprenant une mono-éthanol-amine, une di-éthanol-amine et une tri-éthanol-amine, stripper l'ammoniac du produit de réaction dans un stripper d'ammoniac, moyennant quoi un produit de réaction strippé est obtenu, concentrer le produit de réaction strippé par l'évaporation d'eau dans un évaporateur, moyennant quoi un produit de réaction concentré est obtenu, et amener le produit de réaction concentré à une installation de distillation pour séparer la tri-éthanol-amine de la mono-éthanol-amine et de la diéthanolamine, **caractérisé en ce que** l'ammoniac strippé du produit de réaction est amené à un absorbeur d'ammoniac pour le recyclage de l'ammoniac au réacteur, où une portion du produit de réaction à amener au stripper d'ammoniac est chauffée au moyen d'un échangeur de chaleur avec une vapeur de l'évaporateur, et dans lequel le stripper d'ammoniac est intégré directement avec l'absorbeur d'ammoniac en amenant une vapeur de la fraction de tête du stripper dans un fond de l'absorbeur d'ammoniac.

2. Procédé selon la revendication 1, dans lequel l'absorbeur d'ammoniac fonctionne à une pression au-dessus de 4 bar, de préférence à ou au-dessus de 4,5 bar, selon une préférence particulière à ou au-dessus de 4,8 bar.

3. Procédé selon l'une quelconque des revendications 1 ou 2, dans lequel l'absorbeur d'ammoniac possède une cuve, où la concentration de l'ammoniac dans la cuve est au moins de 40%, selon une préférence particulière d'au moins 42,3%.

4. Procédé selon l'une quelconque des revendications précédentes, dans lequel les couleurs des MEA et DEA quittant l'installation de distillation sont en dessous de 10.

5. Procédé selon l'une quelconque des revendications précédentes, dans lequel le rapport de l'ammoniac à l'éthanol-amine va jusqu'à ou dépasse 6:1 au moment de l'entrée dans le réacteur.

6. Procédé selon l'une quelconque des revendications précédentes, dans lequel le produit de réaction concentré a une température maximale de 180°C avant l'entrée dans l'installation de distillation.

7. Procédé selon l'une quelconque des revendications précédentes, dans lequel la température dans le réacteur est diminuée pour abaisser la concentration des tri-éthanol-amines.

8. Installation de production d'éthanol amines comprenant un réacteur (10), où dans le réacteur, de l'éthylène oxyde (4) est amené à réagir avec de l'ammoniac (5, 105) et de l'eau (6) en un produit de réaction (11) comprenant mono-éthanol-amine, di-éthanol-amine et tri-éthanol-amine, un stripper d'ammoniac (20) pour stripper l'ammoniac du produit de réaction (11), moyennant quoi un produit de réaction strippé (23) est obtenu, un évaporateur (30) pour l'évaporation de l'eau du produit de réaction strippé (23) moyennant quoi un produit de réaction concentré (31) est obtenu, et une installation de distillation (4) pour séparer le produit de réaction concentré (31) contenant la tri-éthanol-amine de la mono-éthanol-amine et de la di-éthanol-diamine, où un absorbeur d'ammoniac (100) est prévu pour le recyclage de l'ammoniac (25) strippé du produit de réaction (11) au réacteur (10), **caractérisée en ce qu'**un échangeur de chaleur (22) est pourvu pour chauffer une portion du produit de réaction strippé (23) à amener au stripper d'ammoniac (20) avec une vapeur de l'évaporateur (30) et dans laquelle le stripper d'ammoniac (20) est intégré directement avec l'absorbeur d'ammoniac au moyen d'un raccordement direct entre les deux.

9. Installation selon la revendication 8, dans laquelle l'échangeur de chaleur comprend un évaporateur à film tombant, un thermosiphon ou un rebouilleur à circuit forcé.

10. Installation selon l'une quelconque des revendications précédentes 8 à 9, où l'installation de distillation (40) comprend une colonne de déshydratation (50) pour séparer l'eau résiduelle du produit de réaction concentré (31), où une première colonne de distillation (60) est prévue pour la séparation de la mono-éthanol-amine de la di-éthanol-amine et de la tri-éthanol-amine, une deuxième colonne de distillation (70) est prévue pour la séparation de la di-éthanol-amine de la tri-éthanol-amine.

11. Installation selon la revendication 10, dans laquelle une troisième colonne de distillation (80) est prévue pour une concentration plus poussée de la tri-éthanol-amine.

12. Installation selon la revendication 11, dans laquelle la troisième colonne de distillation (80) comprend un seul lit d'emballages.

13. Installation selon les revendications 10 ou 11, où la tri-éthanol-amine quittant la cuve de la troisième colonne de distillation (80) comme résidu de tri-éthanol-amine (82) est encore purifiée dans un évaporateur à chemin court (90) en un distillat de tri-éthanol-amine (91) d'une valeur de couleur inférieure à 40 et un résidu de tri-éthanol-amine (92).

14. Installation selon l'une quelconque des revendications 10 à 13, où au moins un deuxième réacteur (200) est prévu pour ajouter encore de l'éthylène oxyde au produit de réaction concentré (31) pour décaler la distribution du produit à des portions plus élevées de la di-éthanol-amine.
